# DEMANDE DE BREVET EUROPEEN

(11) **EP 1 031 326 A1**
(43) Date de publication de la demande: **30.08.2000**
(21) Numéro de dépôt: 00440031.3
(22) Date de dépôt: 03.02.2000
(51) Int. Cl.: A61C 19/00

(54) **Dispositif permettant la photo-activation de matériaux composites photosensibles utilisés notamment dans le domaine dentaire**

(30) Priorité: 05.02.1999 FR 9901657; 20.09.1999 FR 9911860
(71) Demandeur: Decaudin, Jean-Michel, 13880 Velaux (FR)
(72) Inventeur: Lequime, Michel, 13510 Eguilles (FR); Duret, Elisabeth, 11560 Fleury d'Aude (FR); Decaudin, Jean-Michel, 13880 Velaux (FR); Duret, Bernard, 38000 Grenoble (FR)
(74) Mandataire: Ph. Arbousse Bastide

(57) **Abrégé**

Appareil permettant la photo activation de matériaux composites photosensibles utilisés notamment dans le domaine dentaire du type comprenant un boîtier (1) renfermant une unité d'alimentation électrique (11) pilotée électriquement par une unité centrale électronique (10) et une source de lumière (2) reliée à ladite alimentation (11) constituée d'une pluralité de diodes électroluminescentes (20).

Il se caractérise en ce que les diodes (20) sont associées à un dispositif de concentration (3) permettant de concentrer de manière optimale, en un point donné, la lumière issue de toutes les zones émissives desdits émetteurs photoniques (20).

## Description

La présente invention a pour objet un appareil permettant la photo activation de matériaux composites photosensibles utilisés notamment dans le domaine dentaire.

Dans le domaine dentaire les matériaux composites sont constitués d'une résine photopolymérisable réagissant à la lumière par modification de ses couches moléculaires ou par transformation thermique de ses molécules. Ces deux phénomènes qui peuvent se conjuguer sont dépendants de la longueur d'onde du rayonnement émis mais aussi de la capacité d'absorption du matériau composite et ont pour effet d'activer ses photo initiateurs et d'obtenir un corps aux propriétés mécaniques et esthétiques définies en fonction de l'application dentaire.

Les appareils de photopolymérisation sont généralement constitués d'un boîtier renfermant une source émettrice contrôlée par un circuit électronique qui comporte une minuterie permettant d'optimiser le temps d'exposition de manière à minimiser l'échauffement des tissus environnant la zone à traiter. Par ailleurs pour avoir la certitude de recouvrir le domaine spectral de photosensibilité du matériau utilisé et fournir suffisamment d'énergie à la réaction, ces appareils utilisent des lampes, dont le spectre d'émission est très large, en association avec des filtres.

Parmi les différentes sources lumineuses, on a utilisé des lampes à vapeur de mercure, mais celles-ci émettent trop de rayonnements ultraviolets, dangereux pour les yeux du patient.

Pour éviter cet inconvénient ces lampes ont été remplacées par des lampes halogènes. Toutefois ces lampes dont le rapport lumen/watt est faible nécessitent une exposition de la zone à traiter d'une durée importante contraignant le patient à garder longtemps la bouche ouverte. D'autre part cette surexposition s'accompagne d'un échauffement de la lampe qui nécessite l'utilisation de systèmes de refroidissement bruyants et volumineux.

Pour remédier à ces inconvénients on utilise actuellement des lampes à arc Plasma présentant une meilleure efficacité lumen/watt et dont on utilise une partie du spectre entre 430 et 490 nm, ce qui permet d'augmenter significativement l'énergie délivrée et de disposer de la puissance requise pour une polymérisation rapide. Toutefois, cette augmentation de la puissance s'accompagne d'une forte augmentation de la chaleur dégagée.

Dans les appareils connus à ce jour d'une part la conversion de l'énergie électrique en énergie lumineuse est médiocre, et d'autre part les systèmes de refroidissement sont complexes et bruyants, ce qui augmente le coût de l'appareil.

Le document JP07240536 décrit une source lumineuse pour appareil de photopolymérisation constituée d'une part de diodes électroluminescentes émettant un rayonnement compris entre 380 nm et 430 nm et d'autre part d'un dispositif de concentration comprenant des fibres optiques en regard de l'entrée de chacune desquelles est placée l'une desdites diodes.

Toutefois le dispositif de concentration de ce type d'appareil ne permet pas d'obtenir une concentration optimale de la lumière issue des diodes car celles-ci sont munies d'une lentille et que la fibre est au contact de cette lentille, et donc une polymérisation rapide des matériaux du fait d'un mauvais couplage entre lesdites diodes et les fibres optiques.

La présente invention a pour but de remédier à ces inconvénients en proposant un appareil de photopolymérisation de matériaux composites et de produits pour le blanchiment des dents permettant de réaliser une photopolymérisation rapide et graduelle des différentes couches du matériau à traiter tout en permettant de réduire de façon significative la chaleur dégagée.

L'appareil de photopolymérisation selon l'invention est du type comprenant un boîtier renfermant une unité d'alimentation électrique pilotée électriquement par une unité centrale électronique, une source de lumière reliée à ladite alimentation constituée d'une matrice de diodes électroluminescentes et un dispositif de concentration de la lumière issue des diodes et se caractérise essentiellement en ce que :
- dans un premier mode de réalisation le dispositif de concentration permet de juxtaposer en un point de concentration donné la lumière issue de toutes les surfaces émettrices desdites diodes, et est constitué de fibres optiques associées aux diodes électroluminescentes de manière que la surface émettrice d'une diode soit ajustée optiquement au mieux avec la surface d'entrée d'une ou plusieurs fibres optiques correspondantes ;
- dans un deuxième mode de réalisation de l'invention le dispositif de concentration de la lumière issue des diodes permet de superposer en un point donné les images de toutes les surfaces émettrices desdites diodes et est constitué d'une part d'un dispositif de collimation composé d'une pluralité de lentilles convergentes disposées chacune devant la surface émettrice d'une diode électroluminescente de telle sorte que ladite surface émettrice se trouve au foyer objet de la lentille correspondante et que les rayons soient réfractés parallèlement à l'axe de cette dernière, et d'autre part un dispositif de focalisation formé d'une lentille convergente de dimensions appropriées pour permettre de réfracter les rayons incidents parallèles issus du dispositif de collimation vers le point de concentration qui est le foyer de ladite lentille.

Les lentilles de collimation ou de focalisation peuvent être de type réfractive, diffractive (lentilles de Fresnel, lentilles holographiques, binaires), ou à gradient d'indice, ou une composition de ces différents types de lentilles. Les lentilles de focalisation seront de préférence de type réflectif.

Le point donné où se concentre la lumière issue des diodes à la sortie du dispositif de concentration peut être l'entrée d'un guide d'onde muni d'un embout pour appliquer la lumière provenant de la source lumineuse sur la zone à traiter ou être directement la zone à traiter.

Conformément à l'invention les diodes sont ajustées optiquement au mieux avec la ou les fibres correspondantes en positionnant la surface d'entrée de la fibre correspondante en contact avec la surface d'émission de la diode ou à proximité immédiate de cette dernière et en conservant un rapport entre la surface émettrice de la diode et la surface d'entrée de la ou des fibres en regard aussi proche que possible de 1.

L'ajustement optique peut se faire aussi en formant l'image par l'intermédiaire d'une optique d'imagerie de la zone émissive de la diode sur la face d'entrée d'une ou plusieurs fibres en respectant un rapport de surface aussi proche que possible de 1.

Dans un mode de réalisation préférentiel du dispositif de concentration constitué de fibres optiques, les diodes sont disposées régulièrement sur une plaque et les fibres optiques sont insérées chacune dans un trou de diamètre légèrement supérieur au diamètre desdites fibres et pratiqué dans une plaque positionnée par des moyens de centrage à proximité de la plaque support de diodes et dont le pas des trous est égal à celui desdites diodes.

Selon une caractéristique additionnelle de l'invention la commande d'alimentation des diodes électroluminescentes peut être effectuée manuellement au moyen de boutons de réglage ou automatiquement au moyen de l'unité centrale renfermant dans sa mémoire les paramètres inhérents à la commande desdites diodes permettant d'obtenir différents profils énergétiques.

Selon une autre caractéristique additionnelle de l'invention l'alimentation électrique des diodes est réglable en mode pulsée de manière à obtenir des puissances instantanées plus élevées, favorable pour des applications particulières de polymérisation.

Selon une autre caratéristique additionnelle de l'invention la résultante lumineuse en sortie du faisceau de fibres optiques comporte au moins deux longueurs d'ondes se situant préférentiellement respectivement autour de 380 ou 475 µm et 750 µm, de manière à obtenir à la fois un rayonnement nécessaire à certains matériaux composites et un rayonnement adapté pour une action thermique sur les produits destinés aux blanchiment des dents.

Selon une autre caractéristique additionnelle de l'invention le coeur du guide optique dans lequel se propage la résultante lumineuse en sortie du dispositif de concentration peut être réalisé en matière plastique, en verre, ou être liquide.

Les avantages et les caractéristiques de la présente invention ressortiront plus clairement de la description qui suit et qui se rapporte au dessin annexé, lequel en représente plusieurs modes de réalisation non limitatifs.
- la figure 1 représente le schéma synoptique du dispositif selon l'invention.
- la figure 2a représente une vue en perspective de la source lumineuse constituée d'une multiplicité de diodes électroluminescentes associées à un dispositif de concentration constitué de fibres optiques.
- la figure 2b représente une vue de détail du couplage d'une diode avec une fibre optique.
- la figure 3a représente le spectre d'émission de quatre diodes électroluminescentes de composantes spectrales différentes.
- la figure 3b représente le spectre global résultant de l'addition des émissions lumineuses des quatre diodes précdentes.
- la figure 4 représente une vue en perspective schématique du dispositif de concentration dans un autre mode de réalisation.
- la figure 5 représente le dessin optique du dispositif de concentration de la figure précédente.
- la figure 6a représente une forme particulière de la surface émettrice d'une diode électroluminescente.
- la figure 6b représente la forme particulière de la surface émettrice de la figure précédente dans une orientation différente.
- la figure 7 représente la superposition des images au foyer image du dispositif optique.
- la figure 8 représente une source de lumière composée d'émetteurs photoniques comportant des surfaces d'émission de forme identique et selon deux orientations différentes.
- la figure 9 représente une vue en perspective d'une anamorphose d'une image d'une certaine forme.

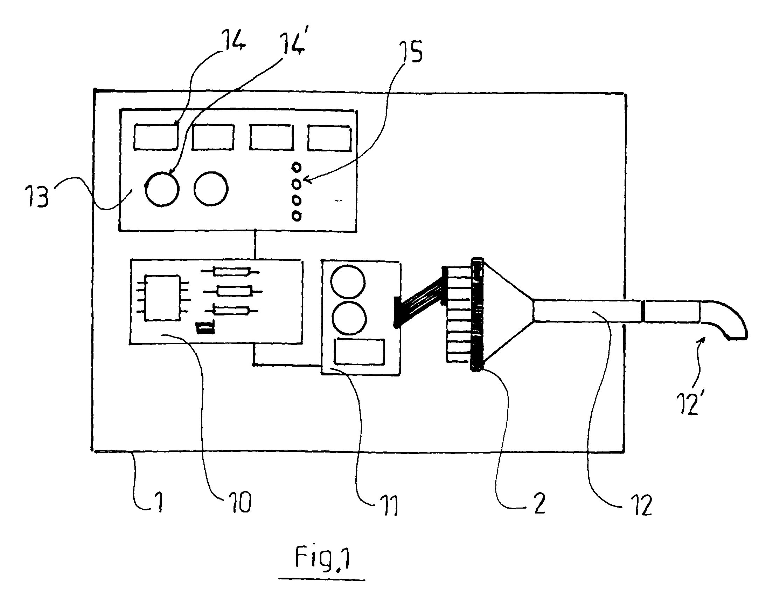

Si on se réfère à la figure 1 on peut voir qu'un appareil destiné à la photopolymérisation de matériaux composites et à la photoàctivation de produits destinés au blanchiment des dents est constitué d'un boîtier 1 abritant une unité centrale électronique 10 reliée électriquement à une unité d'alimentation 11 d'une source lumineuse 2 générant de la lumière destinée à être appliquée au moyen d'un guide d'ondes 12 se terminant par un embout coudé 12', sur une zone à traiter, non représentée.

L'unité centrale 10 est également reliée électriquement à une interface 13 comportant des touches de commande 14 et 14' et des voyants de contrôle 15 permettant à l'opérateur de dialoguer avec l'appareil et notamment de régler les paramètres inhérents à l'émission de la lumière.

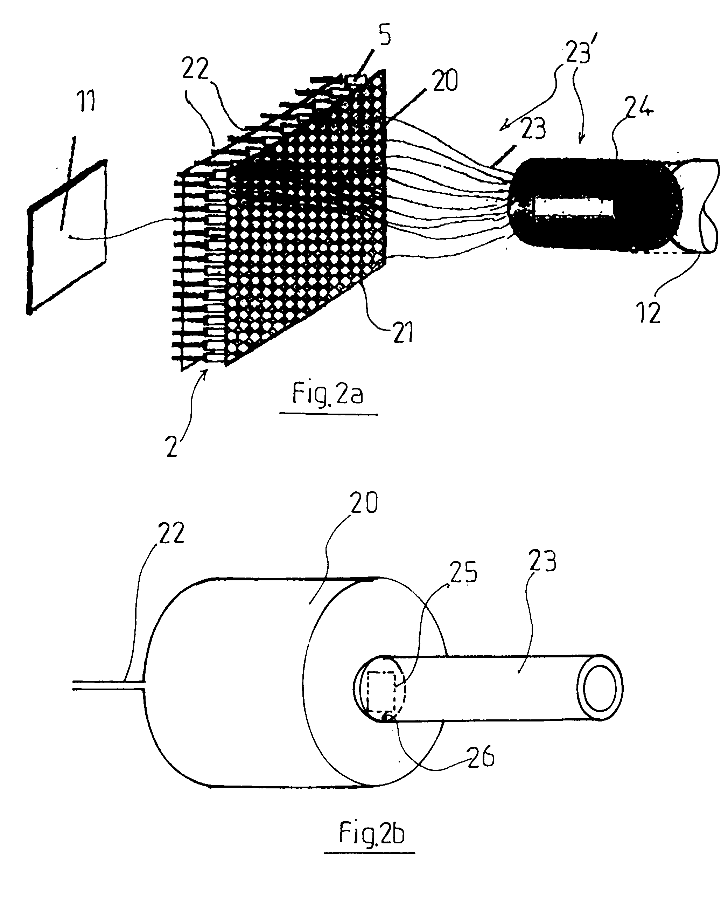

Si on se réfère maintenant à la figure 2a on peut voir que la source lumineuse 2 comprend une pluralité de diodes électroluminescentes 20 associées à un dispositif de concentration 23' et disposées régulièrement sous la forme d'une matrice en étant solidarisées sur un support 21, par exemple par collage, muni de connexions électriques 22 permettant de connecter les diodes 20 à l'unité d'alimentation 11. Le dispositif de concentration 23 permet de concentrer la lumière issue des diodes 20 en un point donné qui peut être l'entrée du guide d'onde 12 ou directement la zone à traiter. Le dispositif de concentration 23' est constitué d'un faisceau de fibres optiques 23, dont pour chaque fibre optique 23 l'une des extrémités est placée et maintenue en regard de la surface émettrice d'une diode électroluminescente 20 de manière à recueillir l'onde lumineuse qu'elle émet.

On peut voir également que les fibres optiques 23 sont réunies et maintenues les unes contre les autres dans une bague 24 permettant d'obtenir, à la sortie du dispositif de concentration 3 formée par l'ensemble des sorties des fibres 23, une surface d'émission de faibles dimensions dont la résultante lumineuse sera acheminée à travers le guide d'ondes 12 qui est sensiblement de même diamètre que le diamètre de la bague 24.

La figure 2b montre une diode électroluminescente 20 comportant une surface d'émission 25 située en regard et à proximité immédiate ou au contact de la surface d'entrée 26 d'une fibre optique 23 couplée à ladite diode 2 et dont les dimensions de la surface d'entrée 26 de la fibre 23 sont sensiblement identiques à celles de la surface 25 d'émission de la diode 20 de manière à recueillir le maximum de la partie active de la surface 25 d'émission de la diode 20.

Les fibres optiques 23 peuvent être insérées dans des trous pratiqués dans une plaque, avec un pas de perçage sensiblement identique au pas de répartition des diodes 20.

Par conséquent :
- d'une part du fait du rapport entre la surface émettrice d'une diode et la surface d'entrée de la fibre en regard,
- et d'autre part du fait du positionnement de la surface d'émission d'une diode au contact ou à proximité immédiate, c'est à dire quasiment au contact, de la surface d'entrée de la fibre en regard procurant un très bon couplage entre les diodes et les fibres, la concentration obtenue à la sortie des fibres 23 est optimale, ce qui permet d'effectuer une polymérisation rapide particulièrement adaptée dans le domaine de la photopolymérisation des matériaux composites dentaires.

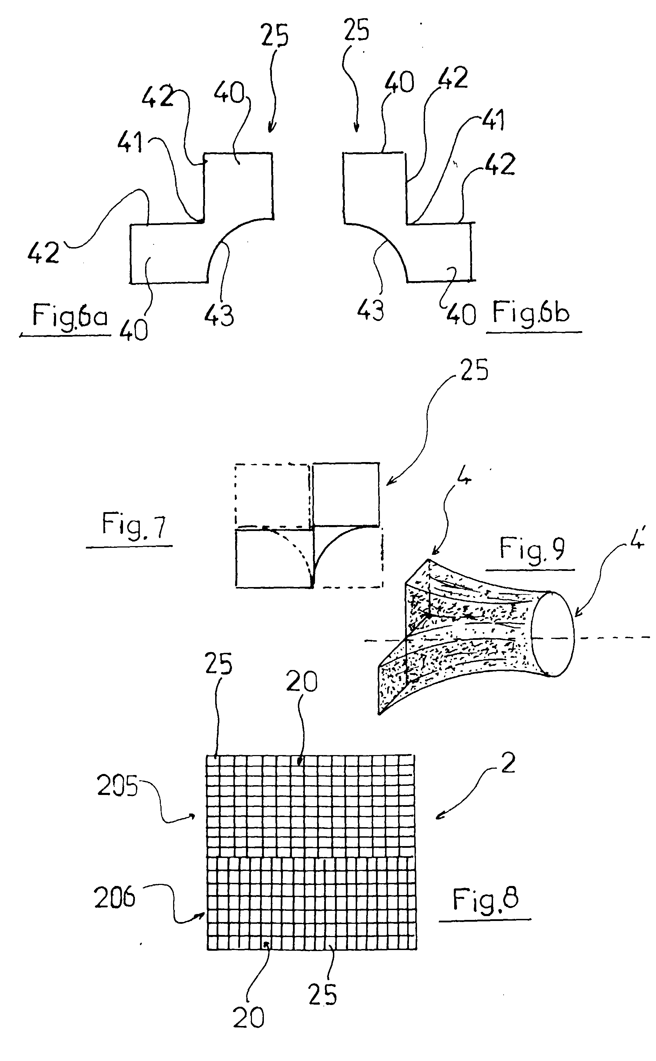

On notera que plusieurs fibres peuvant être associées à une seule diode, notamment lorsque la diode comporte une surface d'émission particulière présentant plusieurs morceaux de surfaces émettrices tels que par exemple deux surfaces carrées juxtaposées (figures 6a et 6b). Dans ce cas chaque morceau de surface émettrice sera alors associé, en respectant les paramètres d'ajustement optique énoncés ci-dessus, à une fibre optique.

Les fibres optiques 23 peuvent être insérées dans les trous pratiqués dans une plaque, avec un pas de perçage sensiblement identique au pas de répartition des diodes 20.

Les diodes électroluminescentes 20 seront de préférence du type sans lentille optique de couplage pour rapprocher au plus près la fibre de la surface émissive de la diode et les fibres optiques 23 seront de préférence des fibres présentant une ouverture numérique importante pour capter la maximum de lumière émise par les diodes électroluminescentes 20 et présentant une atténuation linéique faible dans les domaines de longueur d'onde utilisés.

Le guide d'ondes 12 peut être une fibre optique liquide présentant une perte en ligne très faible et offrant une bonne homogénéisation de la lumière issue des diodes 20 par l'intermédiaire des fibres optiques 23.

Les diodes électroluminescentes 20 peuvent être pilotées électriquement soit manuellement au niveau de l'interface 13 en actionnant des touches 14 prévues à cet effet, soit par l'unité centrale 10 au moyen d'un programme de commande de la puissance des diodes 20 agissant sur l'unité d'alimentation 11 de ces dernières.

De ce fait les diodes 20 peuvent être commandées en alimentation électrique, tout ou rien, individuellement ou par paquet de diodes de même spectre d'émission, de manière à pouvoir modifier le spectre d'émission de la résultante lumineuse à la sortie du toron 24. Il est également possible de modifier le spectre global d'émission en faisant varier l'intensité du courant électrique traversant les diodes 20, cette variation s'accompagnant d'une variation de la puissance émise par la diode 20 qui provoque une variation du spectre global.

Ainsi la variation du courant dans un ensemble de diodes 20 d'un type spectral donné entraînera une modification de la puissance d'émission des diodes 20 et donc une variation du spectre de la résultante lumineuse en sortie du réseau de fibres optiques 23, de même que l'interruption de l'alimentation des diodes de l'un de ses ensembles modifiera, par suppression d'un spectre, les composantes spectrales de la résultante lumineuse en sortie des filtres 23.

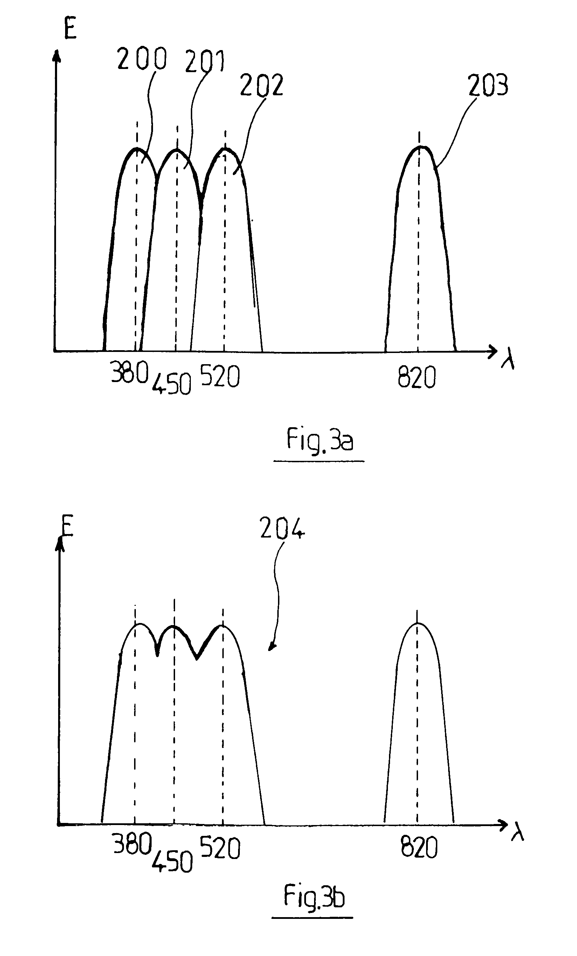

Sur la figure 3a on peut voir quatre courbes 200, 201, 202, 203 qui représentent chacune le spectre d'émission d'un type de diodes électroluminescentes 20 comportant une longueur d'onde respectivement, 380, 450, 520 et 820 nm, pour laquelle l'émission lumineuse est maximale.

Dans ce cas les diodes électroluminescentes 20 dont le spectre se situe autour de 380 nm sont particulièrement adaptées pour le traitement de matériaux plus sensibles aux ultraviolets, tandis que les diodes électroluminescentes dont le spectre se situe autour de 450 nm sont adaptées pour le traitement des matériaux composites de la famille, par exemple, des camphoroquinones et les diodes dont le spectre se situe autour de 820 nm permettent une action thermique sur les produits de blanchiments.

Ainsi le choix des caractéristiques spectrales de la lumière émise par les diodes électroluminescentes permet d'obtenir le profil spectral 204 souhaité (figure 3b) en fonction de l'application, par addition de la lumière émise par les différentes diodes 20. Par conséquent, contrairement aux systèmes actuels ne comportant pas de matrice de diodes électroluminescentes le dispositif selon l'invention permet de d'obtenir le profil spectral souhaité, par addition de lumière, et donc sans filtres, et toute la quantité de lumière émise.

On notera que du fait que les diodes électroluminescentes 20 ont une faible consommation en courant électrique, l'énergie apportée au dispositif peut se faire à partir de batteries rechargeables ou non.

De manière avantageuse les diodes électroluminescentes 2 pourront être solidarisées sur un support interchangeable muni de connecteurs permettant sa connexion rapide à l'appareil selon l'invention.

D'autre part de manière à pouvoir modifier les profils énergétiques mémorisés par le constructeur dans la mémoire morte de l'unité centrale 10 qui pourra être programmé à distance par l'utilisateur au moyen par exemple d'une carte à mémoire du type carte à puce contenant le programme incluant les paramètres inhérents à la commande électrique des diodes.

La programmation à distance du microprocesseur, qui peut être effectuée également au moyen d'un micro-ordinateur via une interface de type modem permet aussi au fabricant de matériaux composites d'optimiser le spectre d'action de la source lumineuse en fonction de son matériau et d'assurer un service après vente ou une mise à jour très rapidement et très simplement.

Des essais ont montré que, pour obtenir une polymérisation rapide souhaitable tant sur le plan clinique que biologique, l'éclairage fourni par la source lumineuse 2 doit se situer entre 1 et 2W/cm2 et préférentiellement autour de 1,5W/cm2. Cet éclairage est directement en rapport avec le nombre de diodes électroluminescentes 20 et la puissance qui leur est fournie par la carte d'alimentation 11.

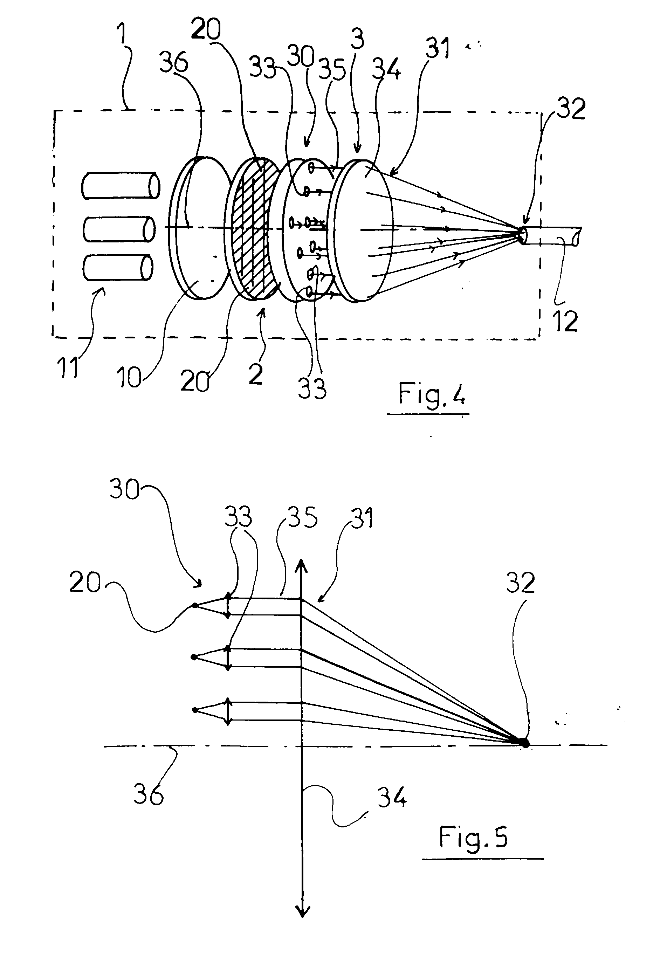

Si on se réfère maintenant aux figures 4 et 5 on peut voir, dans un autre mode de réalisation, un dispositif de concentration 3 constitué d'un dispositif de collimation 30 et d'un dispositif de focalisation 31 permettant de réaliser une collimation des rayons lumineux émis par les diodes électroluminescentes 20 d'une source lumineuse 2 et de concentrer l'ensemble de ces rayons en un point de concentration donné 32 correspondant sensiblement soit directement à la zone de traitement soit à l'entrée du guide d'onde 12.

Le dispositif de collimation 30, qui se trouve en regard des diodes électroluminescentes 20, est constitué d'une matrice de lentilles convergentes 33 définie de manière que ces dernières soient positionnées chacune en regard de la surface émettrice d'une diode lumineuse 20 et à une distance telle que la diode 20 se trouve au foyer objet de la lentille 33 correspondante. Le dispositif de focalisation 31 comporte une lentille convergente 34, parallèle au plan contenant les lentilles 33 et au plan contenant les diodes 20, de dimensions sensiblement égales à celles de la matrice 30 de lentilles 33. La lentille convergente 34 possède un foyer image correspondant sensiblement à l'entrée du guide d'onde 12 où sont réfractés les rayons incidents 35 parallèles à l'axe principal 36 de la lentille convergente 34 et aux axes optiques des lentilles convergentes 31 du dispositif de collimation 30.

Les diodes lumineuses 20 connues actuellement comportent des surfaces émettrices 25 dont la forme peut être simple telle que par exemple un carré ou, comme on peut le voir sur la figure 6a, complexe comportant plusieurs morceaux de surface émettrice telle que par exemple deux surfaces émettrices carrées 40 reliées par un de leurs coins 41 et dont deux côtés adjacents 42, réunis par ce point, forment un arc de cercle 43. La figure 6b montrant le même motif que celui de la figure 3a mais avec une orientation différente, celui-ci étant tourné de 90° par rapport à l'axe de la diode 20 correspondante.

La figure 7 montre la résultante optique au foyer image 32 correspondant à une superposition de motifs correspondants à ceux des figures 6a et 6b et dont certains sont orientés selon la représentation de la figure 6a et d'autres selon la représentation de la figure 6b.

La figure 8 montre une source lumineuse 2 constituée d'une série 205 de diodes électroluminescentes 20 de forme rectangulaire orientées dans une direction et une série 206 de diodes 20 du même type mais orientée qui possèdent chacune une surface d'émission 25 de forme rectangulaire et une série 206 de diodes 20 possédant toutes la même surface d'émission 25 mais orientée différemment, et notamment tournée de 90° par rapport à l'axe d'émission de la diode 20 correspondante.

La source 2 pourra être constituée d'une diode électroluminescente 20 de longueur d'onde différente.

La figure 9 montre que la forme d'une image à la sortie du dispositif de concentration réalisé selon l'un 23' ou l'autre 3 des modes de réalisation décrits ci-dessus peut être modifiée par un dispositif connue de la technique réalisant une anamorphose transformant par exemple une image complexe 4 telle que celle de la figure 6a ou de la figure 6b en une image circulaire 4', le dispositif réalisant cette anamorphose pourrait être disposé entre le foyer image du dispositif de concentration et la surface à traiter ou l'entrée du guide d'ondes.

## Revendications

1. Appareil permettant la photo activation de matériaux composites photosensibles utilisés notamment dans le domaine dentaire du type comprenant un boîtier (1) renfermant une unité d'alimentation électrique (11) pilotée électriquement par une unité centrale électronique (10), une source de lumière (2) reliée à ladite alimentation (20) constituée d'une matrice de diodes électroluminescentes et un dispositif de concentration (23') de la lumière issue desdites diodes (20) caractérisé en ce que ledit dispositif de concentration (23') permet de juxtaposer en un point donné de concentration la lumière issue de toutes les surfaces émettrices (25) desdites diodes (20) et est constitué de fibres optiques (23) associées aux diodes électroluminescentes (20) de manière que la surface émettrice (25) d'une diode (20) soit ajustée optiquement au mieux avec la surface d'entrée d'une ou plusieurs fibres optiques (23).

2. Appareil selon la revendication 1 caractérisé en ce que les diodes (20) sont disposées régulièrement sur une plaque et les fibres optiques (23) sont insérées chacune dans un trou de diamètre légèrement supérieur au diamètre desdites fibres (23) et pratiqué dans une plaque positionnée par des moyens de centrage à proximité de la plaque support de diodes (20) et dont le pas des trous est égal à celui desdites diodes.

3. Appareil selon la revendication 1 ou la revendication 2 caractérisé en ce qu'une optique d'imagerie image la zone émissive de la diode électroluminescente sur la face d'entrée d'une ou plusieurs fibres et que la surface de ces fibres couvre cette image dans un rapport aussi proche que possible de 1.

4. Appareil permettant la photo activation de matériaux composites photosensibles utilisés notamment dans le domaine dentaire du type comprenant un boîtier (1) renfermant une unité d'alimentation électrique (11) pilotée électriquement par une unité centrale électronique (10), une source de lumière (2) reliée à ladite alimentation (11) constituée d'une matrice de diodes électroluminescentes (20) et un dispositif de concentration (3) de la lumière issue desdites diodes (20) caractérisé en ce que le dispositif de concentration (3) permet de superposer en un point de concentration donné (32) les images de toutes les surfaces émettrices (25) desdites diodes (20) et est constitué d'une part d'un dispositif de collimation (30) composé d'une pluralité de lentilles convergentes (33) disposées chacune devant la surface émettrice (25) d'une diode électroluminescente (20) de telle sorte que ladite surface émettrice (25) se trouve au foyer objet de la lentille (33) correspondante et que les rayons soient réfractés parallèlement à l'axe de cette dernière, et d'autre part un dispositif de focalisation (31) formé d'une lentille convergente (34) de dimensions appropriées pour permettre de réfracter les rayons incidents (35) issus du dispositif de collimation (30) vers le point de concentration (32) qui est le foyer de ladite lentille convergente (34).

5. Appareil selon l'une quelconque des revendications précédentes caractérisé en ce que la commande d'alimentation des diodes électroluminescentes (20) peut être effectuée manuellement au moyen de boutons de réglage (14, 14') ou automatiquement au moyen de l'unité centrale (10) renfermant dans sa mémoire les paramètres inhérents à la commande desdites diodes (20) permettant d'obtenir différents profils énergétiques.

6. Appareil selon l'une quelconque des revendications précédentes caractérisé en ce que l'alimentation électrique des diodes (20) est réglable en mode pulsé de manière à obtenir des puissances instantanées plus élevées.

7. Appareil selon l'une quelconque des revendications précédentes caractérisé en ce que le coeur du guide optique dans lequel se propage la résultante lumineuse en sortie du dispositif de concentration (23', 3) peut être réalisé en matière plastique, en verre, ou être liquide.

8. Appareil selon l'une quelconque des revendications précédentes caractérisé en ce que la surface émettrice (25) des diodes électroluminescentes (20) a la forme d'un carré ou d'un rectangle ou une forme complexe.

9. Appareil selon la revendication 8 caractérisé en ce que la surface émettrice (25) d'une diode (20) est composée de deux carrés (40) reliés par un de leurs coins (41) et dont deux côtés adjacents (42), réunis par ce point, forment un arc de cercle (43).

10. Appareil selon la revendication 8 ou la revendication 9 caractérisé en ce que la source lumineuse (2) est constituée d'un ensemble (21) de diodes électroluminescentes (20) comportant chacune une surface émettrice (25) de forme identique et d'un ensemble (22) de diodes électroluminescentes (20), de même nombre, comportant chacune une surface émettrice (25) de même forme que celle de la surface émettrice (25) des diodes (20) de la série (21) précédente mais avec une orientation différente.

11. Appareil selon la revendication 10 caractérisé en ce que les surfaces émettrices (25) des diodes (20) de l'un des deux ensembles sont tournées de 90° par rapport aux surfaces émettrice (25) des diodes (20) de l'autre ensemble.

12. Appareil selon l'une quelconque des revendications précédentes caractérisé en ce qu'il comprend un dispositif réalisant une anamorphose de l'image formée de la superposition des images des surfaces émettrices des diodes (20) à la sortie du dispositif de concentration (23', 3).

13. Appareil selon l'une quelconque des revendications précédentes caractérisé en ce que le point de concentration à la sortie du dispositif de concentration (23', 3) est soit l'entrée d'un guide d'onde (12) soit directement la zone à traiter.

14. Appareil selon l'une quelconque des revendications 4 à 13 caractérisé en ce que les lentilles de collimation ou de focalisation sont de type réfractive, diffractive, ou à gradient d'indice, ou une composition de ces différents types de lentilles.

15. Appareil selon l'une quelconque des revendications 4 à 14 caractérisé en ce que les lentilles de focalisation sont de type réflectif.
